# EUROPÄISCHE PATENTSCHRIFT

(11) **EP 0 560 157 B1**
(45) Veröffentlichungstag und Bekanntmachung des Hinweises auf die Patenterteilung: **12.09.2001**
(21) Anmeldenummer: 93103212.2
(22) Anmeldetag: 01.03.1993
(51) Int. Cl.: B01J 8/06, B01J 8/02

(54) **Festbettreaktoren mit kurzem Katalysatorbett in Strömungsrichtung**
Fixed bed reactors with short catalyst bed in the direction of flow
Réacteurs à lit fixe avec lit de catalyseur court dans la direction d'écoulement

(30) Priorität: 12.03.1992 DE 4207905
(43) Veröffentlichungstag der Anmeldung: 15.09.1993
(73) Patentinhaber: BAYER AG, 51368 Leverkusen (DE)
(72) Erfinder: Langer, Reinhard, Dr., W-4150 Krefeld (DE); Buysch, Hans-Josef, Dr., W-4150 Krefeld (DE); Wagner, Paul, Dr., W-4000 Düsseldorf 13 (DE)

(56) Entgegenhaltungen:
- DE-C- 942 805
- FR-A- 1 296 614
- FR-A- 1 297 766
- US-A- 2 078 947
- US-A- 2 433 670
- US-A- 3 128 242

## Beschreibung

Die Erfindung betrifft Festbettreaktoren zur Umsetzung von gasförmigen Edukten oder Eduktgemischen, die dadurch gekennzeichnet sind, daß die Edukte oder Eduktgemische ein flächig ausgebildetes Katalysatorbett annähernd oder gänzlich senkrecht zu diesen Flächen durchströmen.

Hierbei ist es möglich, die Funktion, welche die Temperatur und die Eduktbelastung in Abhängigkeit vom Ort im Katalysatorbett beschreibt, möglichst flach verlaufen zu lassen, wodurch partielle Überlastungen und - speziell bei stark exothermen Reaktionen - Temperaturspitzen zu vermeiden.

Diese Charakteristiken der erfindungsgemäßen Reaktoren ergeben optimale Selektivitäten und Katalysatorstandzeiten.

Die Erfindung betrifft ferner die Verwendung solcher Festbettreaktoren für die Umsetzung von gasförmigen Edukten beziehungsweise Eduktgemischen.

Die katalytische Umsetzung von Gasen an stationären Kontakten läßt sich leicht als kontinuierlicher Prozeß führen und besitzt die reizvolle Annehmlichkeit, daß das Produkt im allgemeinen nicht von Hilfsstoffen, wie Lösungsmitteln oder Katalysatoren, abzutrennen ist. Dementsprechend werden in der chemischen Industrie bevorzugt Chemikalien mit mittlerer und großer Produktionsmenge auf diesem Weg erzeugt.

Viele organische Verbindungen sind im gasförmigen Aggregatzustand bedeutend stabiler als im flüssigen, da aufgrund der geringen räumlichen Konzentration bi- und höhermolekulare Nebenreaktionen unterdrückt werden, so daß beispielsweise stark exotherme Reaktionen auf einem höheren Temperaturniveau durchgeführt werden können als im flüssigen Zustand, was wiederum für eine gekoppelte Wärmeenergiegewinnung von Vorteil ist.

Andere Reaktionen, beispielsweise endotherme Spaltreaktionen, benötigen besonders hohe Temperaturen und können, insbesondere bei empfindlichen Edukten und Produkten, fast nur in der Gasphase durchgeführt werden.

In der Literatur ist eine Vielzahl von Reaktortypen beschrieben (Ullmanns, Enzyklopädie der technischen Chemie, 4. Aufl., Bd. 3, S. 468-649; Kirk-Othmer, Encyclopedia of Chemical Technology, Vol. 19 (1982), S. 880-914). Man unterscheidet dabei Reaktoren mit gelenktem und mit ungelenktem Temperaturprofil.

Reaktoren mit ungelenktem Temperaturprofil bestehen aus einer Katalysatorschüttung beziehungsweise aus einem aus katalytisch aktiven Formkörpern gebildeten Katalysatorbett in einem mehr oder weniger gut isolierten Gefäß von einfacher Geometrie.

Die Reaktionsenthalpie wird in solchen Reaktoren dem Produktgemisch mitgegeben; daher bezeichnet man diese Konstruktionen als adiabatisch. Der Hauptreiz dieser Reaktoren besteht in ihrem einfachen und damit preiswerten und wartungsfreundlichen Aufbau. Bei der Verwendung solcher adiabatischer Reaktoren mit ungelenktem Temperaturprofil ist jedoch zu berücksichtigen, daß chemische Umwandlungen sowohl in kinetischer als auch in thermodynamischer Hinsicht einen exponentiellen Charakter haben und damit sehr empfindlich auf Temperaturänderungen reagieren. Das heißt, daß viele Reaktionen nur in einem engen Temperaturbereich mit genügender Selektivität und Geschwindigkeit ablaufen. Auch der Katalysator selbst kann sich als temperaturempfindlich erweisen.

Da nun die meisten chemischen Reaktionen im Verhältnis zur Wärmekapazität der Systeme eine deutliche Wärmetönung besitzen, kann nur in sehr wenigen Fällen auf die Ab- beziehungsweise Zufuhr von thermischer Energie zur Einhaltung des erforderlichen Temperaturbereichs verzichtet werden (gelenktes Temperaturprofil).

Bei den Reaktoren mit gelenktem Temperaturprofil unterscheidet man die kontinuierliche von der stufenweisen Thermostatisierung beziehungsweise Wärmeabfuhr oder Wärmezufuhr. Die stufenweise Thermostatisierung zerlegt im Prinzip einen adiabatischen Reaktor in Teilabschnitte, nach deren Verlassen das Gas in thermische Wechselwirkung mit einem Wärmeträger gebracht wird oder frische Eduktmischung mit passender Temperatur zugemischt bekommt (Ullmann, loc. cit., S. 473). Die Reaktion darf bei diesem Reaktortyp thermisch nicht zu anspruchsvoll sein, da sonst eine sehr große Stufenzahl erforderlich ist, um die Temperaturgrenzen einzuhalten. Andererseits sind speziell für die Ammoniak- und Methanol-Synthese sehr komplizierte Reaktorkonstruktionen zur optimalen Unterbringung und zum optimalen Betrieb einer größeren Zahl von Katalysatorbettabschnitten und Wärmeaustauschern in einem Hochdruckraum entwickelt worden (EP 297 474, EP 332 757 und darin genannte Literatur).

Für Reaktionen mit extrem starker Wärmetönung und Katalysatoren beziehungsweise Reaktionen mit extrem empfindlichem Temperaturverhalten ist daher eine kontinuierliche Thermostatisierung einzurichten. Hierbei kann man den Katalysator entweder zwischen den Rohren oder in den Rohren eines Wärmetauschers unterbringen (Linde-Reaktor gemäß DE-OS 34 14 717 beziehungsweise Rohrbündelreaktor gemäß Chemie-Ingenieur-Technik 51 (1979), S. 257-265). Reaktoren dieser Art mit Rohrdurchmessern von einem bis zu mehreren cm und Rohrlängen von 2-20 m sind seit langem Stand der Technik. Trotz des ständigen Wärmeflusses in radialer Richtung bildet sich bei stark exothermen Reaktionen ein "hot-spot" in den Reaktorrohren aus, welcher für Selektivitätsverluste oder Katalysatorschädigungen verantwortlich zu machen ist. Es ist bereits beschrieben, den "hot-spot" zu verkleinern, indem der Katalysator zu Beginn der Schüttung mit inertem Material verdünnt wird oder indem die Reaktorrohre am Einströmende einen geringeren Durchmesser besitzen als am Ausströmende (DE-OS 29 29 300; GB 2.132.111). Es ist weiterhin beschrieben, den Mangel an einheitlicher Thermostatisierung durch eine Verkürzung der Rohre des Rohrbündelreaktors auf 1-30 cm, bevorzugt 5-20 cm, zu beheben (DE-OS 36 12 213, EP 244 632). Die Rohre eines solchen Kurzrohrreaktors besitzen Innendurchmesser von 0,5-3 cm, bevorzugt 1-2 cm. Die Vorteile dieses Kurzrohrreaktors werden wie folgt beschrieben:

Große spezifische Kühlfläche und gleichmäßige seitliche Anströmung der Rohre durch den Wärmeträger führen zu definierter Temperaturverteilung im Reaktorrohr, gleichmäßige Queranströmung aller Rohre durch den Wärmeträger soll zu kompakter Bauweise mit maximalem Wärmeübergang führen; auch bei feinkörnigem Katalysator soll es nur einen geringen Druckabfall in den Rohren geben.

In der genannten EP 244 632 wird ferner eine Verbindung zwischen Rohrlänge und Verweilzeit geknüpft, ohne auf diese näher einzugehen; das kurze Rohr mit kurzer Verweilzeit und guter Wärmeabfuhr wird als günstig für stark exotherme Reaktionen mit hoher Aktivierungsenergie und thermisch instabilen Reaktanden und Produkten beschrieben. Hiebei wird jedoch nicht auf die Zahl der Rohre eingegangen, die ein Kurzrohrbündelreaktor beherbergen muß, dessen Rohrlänge um den Faktor 50-500 unter der eines gängigen Reaktors liegt, um technisch relevante Produktmengenströme zu ermöglichen. Zur Substitution eines in der Technik nicht selten anzutreffenden Reaktors mit 10.000 Rohren üblicher Länge wären dies beim Kurzrohrbündelreaktor 500.000 bis 5 Mio Kurzrohreinbauten. Ebenso wenig wird auf die gleichmäßige Durchströmung der riesigen Rohrzahl eingegangen, noch auf die Art, wie dies gewährleistet werden soll, noch auf die Art, wie die Rohre befüllt werden sollen. Die genannte EP 244 632 befaßt sich ausschließlich mit der Verweilzeit und der gleichmäßigen Thermostatisierung, da nach der bekannten Lehre die Aufströmbelastung eines Katalysatorbettes (1 Eduktmischung pro m² Aufströmoberfläche des Katalysatorbettes und Stunde) im allgemeinen keine besonders charakteristische Größe für die Eigenschaften einer heterogenen katalysierten Gasphasenumsetzung ist. Aus FR-A-1 297 766 ist ein Reaktor bekannt, dessen Reaktionsraum in Form dünner Schichten ausgebildet ist, die von kalten Edukten senkrecht durchströmt werden; die heißen Produkte geben an spiralförmig ausgebildeten Wärmeaustauschern einen Teil der Reaktionswärme ab.

Die Erfindung betrifft Reaktoren für die kontinuierliche Umsetzung gasförmiger Stoffe an Festbettkatalysatoren bei kontinuierlicher Temperaturlenkung mit Hilfe eines Wärmeträgers, die gekennzeichnet sind durch die Ausbildung des Katalysatorbettes in Form von einer oder mehreren regelmäßig geformten flächigen Schichten mit einer Dicke von 0,01-50 cm, wobei die Oberfläche der Katalysatorschichten durch eine für Gase durchlässige Schicht abgeschlossen ist und wobei diese Oberfläche auf der Eduktanströmseite und/oder auf der Produktaustrittsseite in einem Abstand von 0,1-10 cm einer in gleicher Weise regelmäßig geformten Wandung gegenüber steht, die den Raum für die umzusetzenden Stoffe beziehungsweise umgesetzten Stoffe vom Raum für den Wärmeträger trennt, und gekennzeichnet sind durch eine solche Führung der umzusetzenden Stoffe, daß diese das Katalysatorbett annähernd oder gänzlich senkrecht zu den flächigen Katalysatorschichten durchströmen.

Die Erfindung betrifft weiterhin den Einsatz der erfindungsgemäßen Reaktoren in Verfahren zu katalysierten, thermogelenkten Gasphasenreaktionen mit hoher endothermer oder exothermer Wärmetönung.

Die beigefügten Zeichnungen zeigen den prinzipiellen Aufbau und die prinzipielle Wirkungsweise der erfindungsgemäßen Reaktoren und einige denkbare technische Ausführungsvarianten.

Die erfindungsgemäßen Reaktoren erlauben es, die Funktion, welche die Temperatur und die Eduktbelastung in Abhängigkeit vom Ort im Katalysatorbett beschreibt, möglichst flach verlaufen zu lassen. Hierdurch werden partielle Überbelastungen und - speziell bei stark exothermen Reaktionen - Temperaturspitzen vermieden. Damit wird es möglich, Nebenreaktionen durch Katalysatorüberlastung oder durch ungeeignete Katalysatortemperatur zu unterdrücken, was sowohl der Selektivität der Produktherstellung als auch der Katalysatorstandzeit zugute kommt. Diese Vorteile werden im erfindungsgemäßen Reaktor dadurch erreicht, daß das Katalysatorbett in Strömungsrichtung eine besonders geringe Ausdehnung erhält und zum anderen dadurch, daß die Reaktionswärme parallel, antiparallel oder parallel und antiparallel zur Strömungsrichtung der Stoffe durch das Katalysatorbett aus der Reaktionszone abgeführt oder der Reaktionszone zugeführt wird. Ein weiterer günstiger Effekt der geringen Durchdringungstiefe solcher gleichmäßig temperierter und belasteter Katalysatorbetten ist ihr geringes Rückhaltevermögen für schwerflüchtige Nebenprodukte, was zusätzlich extrem lange Katalysatorstandzeiten fördert. Im folgenden wird hauptsächlich auf eine Thermostatisierung durch einen sekundären Wärmekreislauf als einer wichtigen technischen Ausführungsform eingegangen; es ist dennoch grundsätzlich möglich, auch eine direkte Wasserdampf-Thermostatisierung oder eine andere vergleichbare direkte Thermostatisierung in den erfindungsgemäßen Reaktoren durchzuführen.

Die erfindungsgemäßen Festbettreaktoren erlauben eine gleichmäßige Belastung und Thermostatisierung des eingesetzten Katalysators. Ein erfindungsgemäßer Reaktor für großtechnische Anwendungen beherbergt trotzdem nur eine geringe Anzahl von Einbauten, die ferner preiswert und leicht zu warten sind.

In den beigefügten Zeichnungen bedeuten die Pfeile mit durchgezogenen Linien und der Bezugsziffer (1) den jeweiligen Stoffstrom von der Zuführung des Edukts (1a) (Ausgangsstoff/-stoffgemisch, gegebenenfalls gemeinsam mit einem Verdünnungs- oder Trägergas) zur Abführung des Produkts (1b) (Reaktionserzeugnis/Reaktionsgemisch, gegebenenfalls mit Verdünnung/Trägergas). Die Pfeile mit gestrichelten Linien und der Bezugsziffer (2) bedeuten den Wärmestrom vom Katalysator zum Wärmeträger. In den beigefügten Zeichnungen zeigen gestrichelte Pfeile zur Illustrierung der Wärmeströme am Katalysator den Fall einer exothermen Reaktion. Endotherme Umsetzungen besitzen einen zur Pfeilrichtung diametralen Wärmefluß; solche Verhältnisse sind in den Zeichnungen explizit nicht berücksichtigt. Die punktierten, schmalen Flächen (3) bedeuten das durch eine für Gase durchlässige Schicht abgeschlossene Katalysatorbett; die für Gase durchlässige Schicht ist in den Zeichnungen nicht besonders hervorgehoben. Die waagerecht gestrichelten Flächen (4) bedeuten den durch eine Wandung abgeschlossenen Raum für den Wärmeträger. Der Wärmeträger gilt in einer dem Fachmann bekannten Weise als durch den erfindungsgemäßen Reaktor bewegt und als mit einer externen Heizung/Kühlung für die Temperaturlenkung endothermer/exothermer Reaktionen verbunden. Wo es in den Zeichnungen beispielhaft gezeigt werden soll, wird der Wärmeträgerstrom durch Pfeile mit Doppelstrich und den Ziffern (4a) für den Eintritt beziehungsweise (4b) für den Austritt bezeichnet.

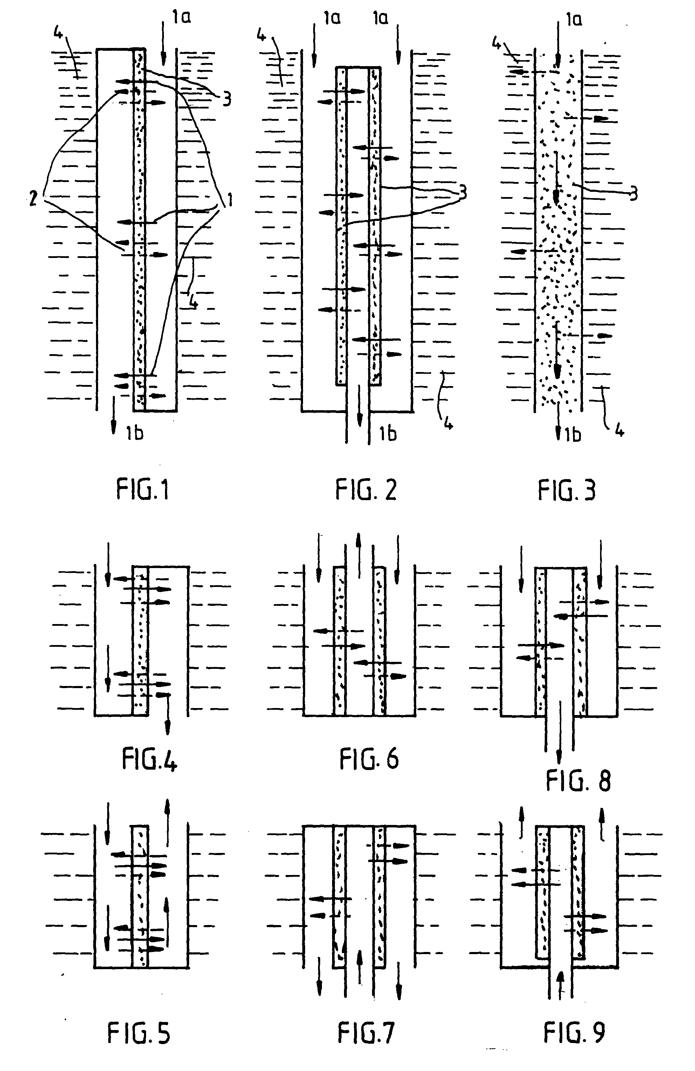

Figur 1 zeigt das Prinzip des erfindungsgemäßen Reaktors am Schnitt durch ein ebenes, flaches Katalysatorbett mit gegenüber liegenden Wärmeträgerräumen; Figur 2 zeigt das Prinzip am Schnitt durch ein als Hohlzylinder ausgebildetes Katalysatorbett; Figur 3 zeigt im Gegensatz hierzu einen Rohrreaktor gemäß Stand der Technik, ebenfalls im Schnitt. Während beim erfindungsgemäßen Reaktor Stoff- und Wärmestrom stets parallel, antiparallel oder sowohl parallel als auch antiparallel zueinander verlaufen, stehen im herkömmlichen Reaktor Stoff- und Wärmestrom orthogonal zueinander. Folgerichtig sind im erfindungsgemäßen Reaktor die von Stoff- und Wärmestrom durchströmten Flächen stets identisch, während sie dies im herkömmmlichen Reaktor nicht sein können.

Figuren 4 - 9 zeigen Variationen von Figur 1 und Figur 2, bei denen in Abwandlung der Bauausführung - flaches Katalysatorbett oder Hohlzylinder als Katalysatorbett beziehungsweise Zu- und Abfuhr von Edukt und Produkt beziehungsweise Notwendigkeit der Energiezufuhr oder Energieabfuhr (bei endothermer oder exothermer Reaktion) - eine parallele oder antiparallele Führung von Edukt-zu Produktstrom in den Verteilungs- und Sammelschichten und eine parallele, antiparallele oder sowohl parallele als auch antiparallele Stoff- zu Wärmefluß-Führung im Katalysatorbett gezeigt wird. Als Wärmefluß ist hierbei derjenige zu verstehen, der vom Katalysatorbett zur Wandung mit dem Wärmeträgerraum oder in umgekehrter Richtung strömt. Die zusätzliche Ziffernbezeichnung der Zeichnungssymbole wurde der Übersichtlichkeit halber in den Figuren 4 -9 fortgelassen. Es ist ersichtlich, daß die Figuren 4 und 5 der Figur 1 zugeordnet sind und die Figuren 6 -9 der Figur 2. In den Figuren 4 und 5 bewegt sich der Wärmestrom sowohl parallel als auch antiparallel zum Stoffstrom; in den Figuren 6 - 9 ist dies entweder parallel oder antiparallel.

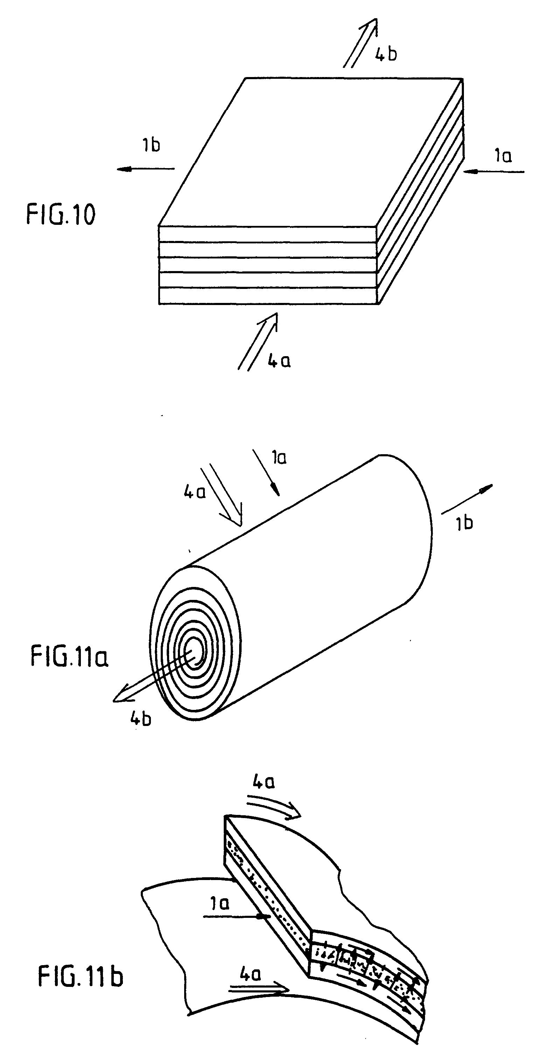

Das Katalysatorbett kann in einer oder mehreren flächigen Schichten ausgebildet werden. So können die flächigen Katalysatorschichten und die gegenüber stehenden, in gleicher Weise regelmäßig geformten Wandungen der Wärmeträgerräume in einer paketähnlichen Anordnung vereinigL werden (Figur 10); die Kombination aus flächigem Katalysator und Wärmeträgerraum kann jedoch auch in einer "aufgerollten" zylindrischen Form angeordnet werden (Figur 11a). Wenn hierbei in der Gesamtanordnung Stoff- und Wärmeträgerfluß auch in orthogonaler Richtung zueinander stehen, trifft dies in der oben geschilderten Weise für das Katalysatorbett im einzelnen nicht zu, wie in Fig. 11b gezeigt wird; in Fig. 11b wird der Wärmeträger im Sinne einer Spirale gelenkt und thermostatisiert sowohl die Edukt- als auch die Produktseite.

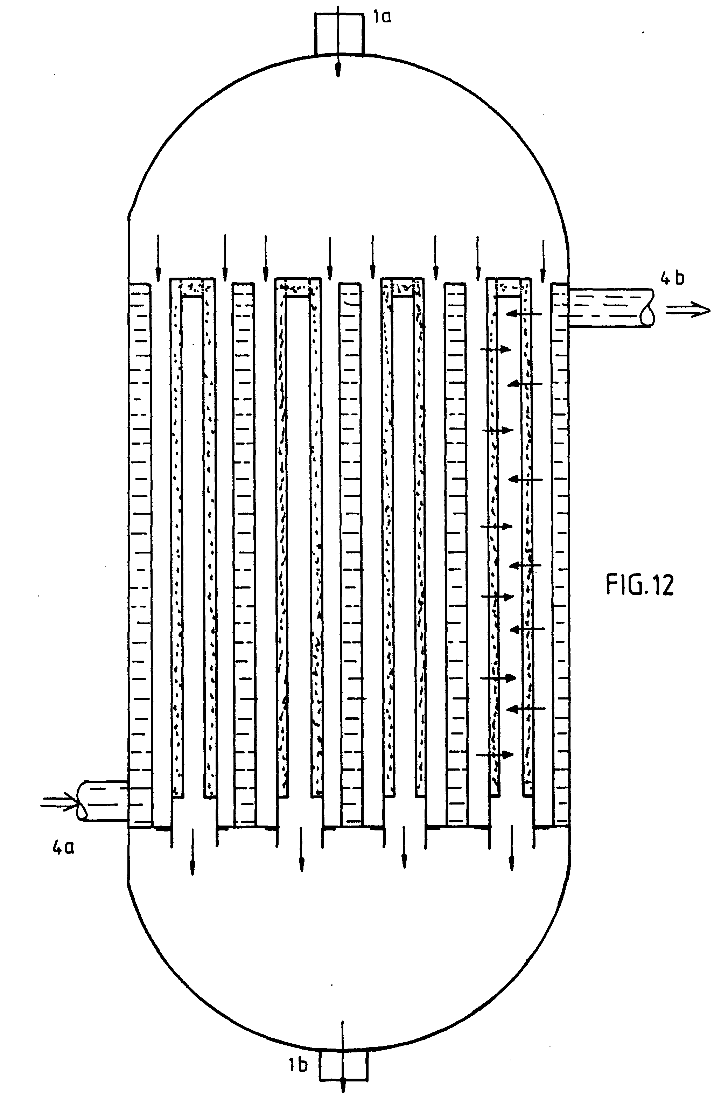

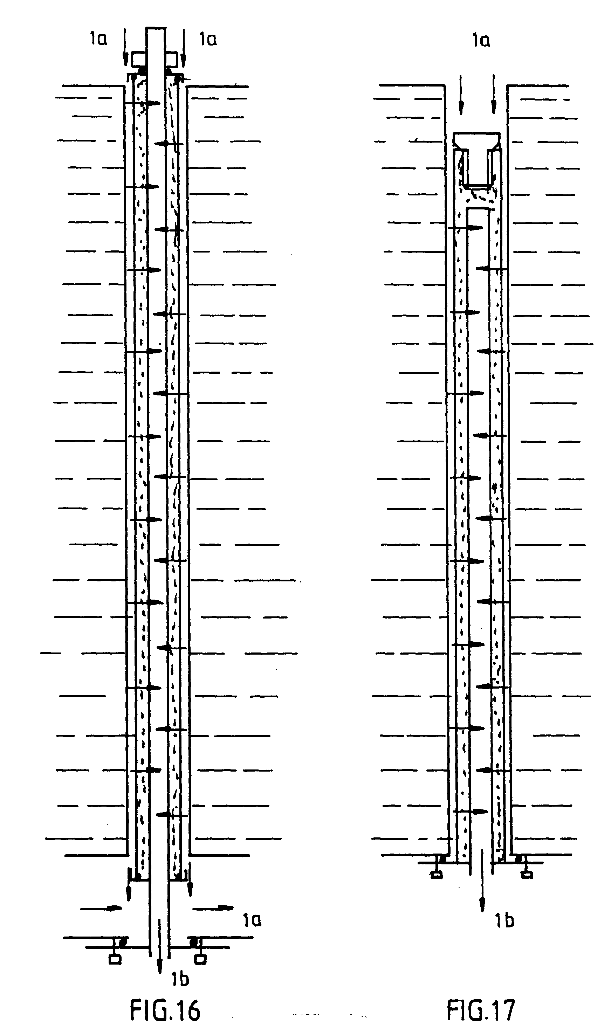

Die Figuren 12 - 15 zeigen mögliche technische Ausführungen, bei denen Rohreinsätze in der Art herkömmlicher Rohrbündelreaktoren Verwendung finden. Die Figuren 16 -19 zeigen einzelne Rohreinsätze für solche Reaktoren, die eingeschraubt, eingeschweißt oder auf andere, grundsätzlich bekannte Weise in den Reaktorkörper eingesetzt werden können.

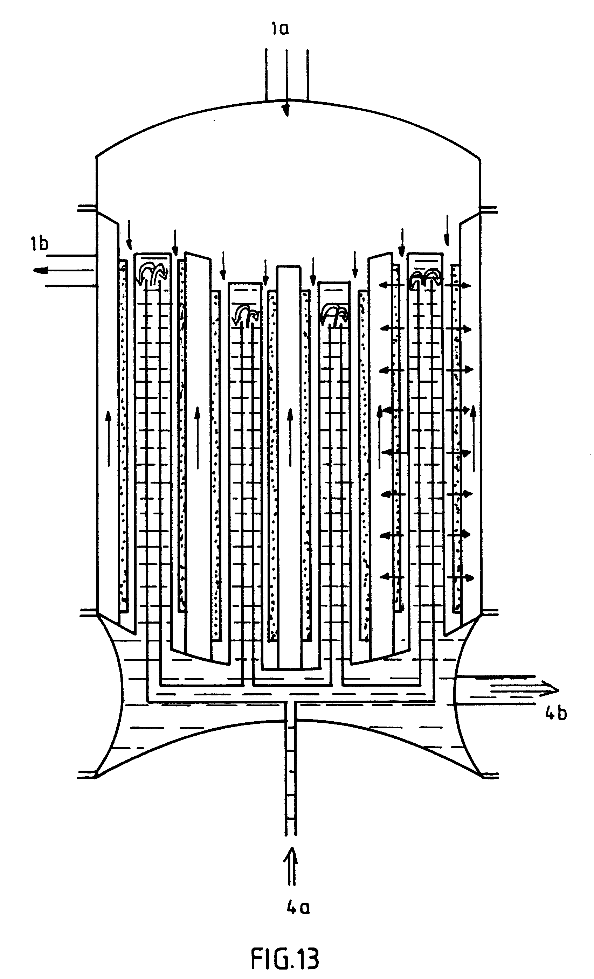

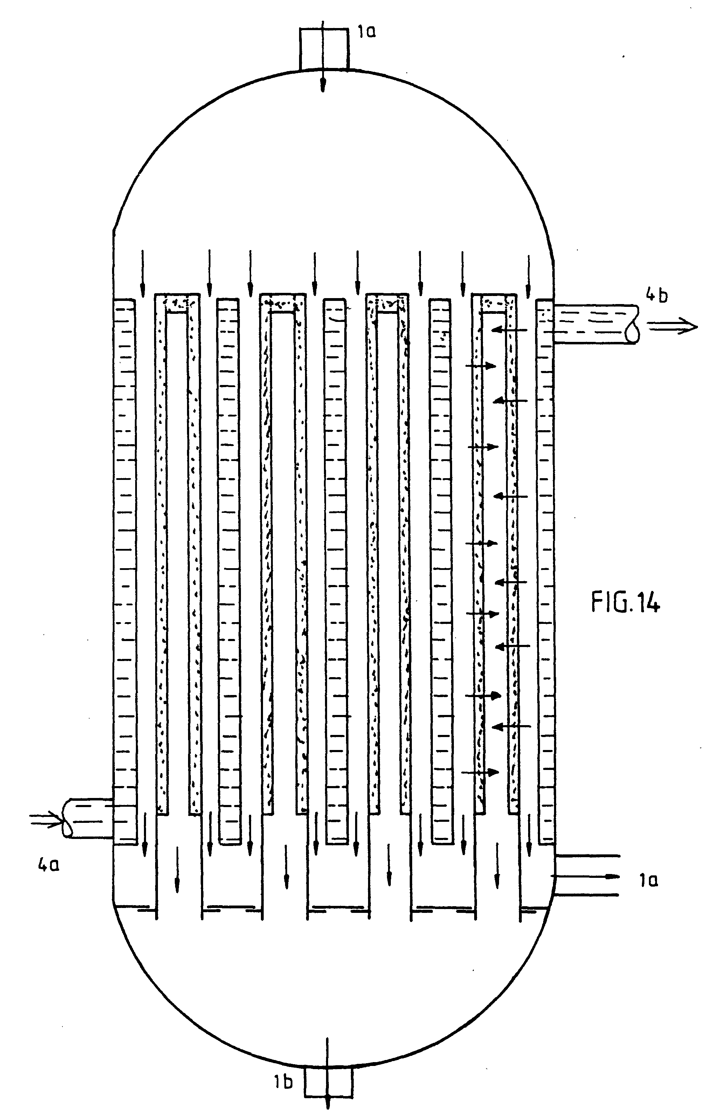

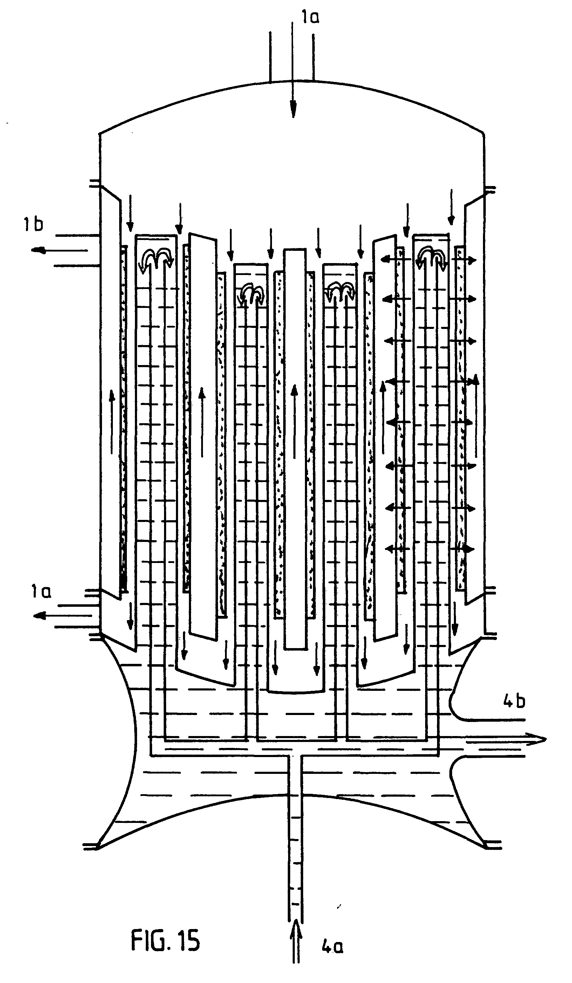

Technische Ausführungsformen der erfindungsgemäßen Reaktoren können auch für einen teilweisen Umlauf der Reaktionspartner um den Reaktor (Rezirkulation) in der Weise ausgelegt werden, daß ein Teil des Reaktorablaufs als Produktstrom entnommen wird, der der Aufarbeitung zugeführt wird, während ein weiterer Teil des Reaktorablaufs erneut an den Reaktoreingang zurückgeführt wird, um dort zur Vervollständigung der Reaktion erneut eingesetzt zu werden oder als systemeigene Verdünnung für die Edukte zu dienen. In entsprechender Weise kann auch ein Teil der Edukte, der noch nicht durch die Katalysatorschicht getreten ist, rezirkulieren; letzteres ist in Figur 14 und 15 anhand der Reaktoren aus den Figuren 12 und 13 gezeigt. Die teilweise Rückführung des Produktstromes und besonders des Eduktstromes in den Reaktoreingang ist gleichzeitig dazu geeignet, die Strömungsgeschwindigkeit des Eduktstromes zwischen der Wärmeaustauscherfläche und der Katalysatoroberfläche zu erhöhen, wobei der Wärmeübergang zwischen Katalysatorbett und Thermostatisiermedium beziehungsweise umgekehrt verbessert wird. In besonderer Weise ist die Eduktrückführung dazu geeignet, weil sich der zirkulierende Eduktstrom entlang der gesamten "Rohrlänge" dem durch den Katalysator tretenden Eduktstrom als konstanter Betrag zuaddiert, was bei der Produktrezirkulation nicht der Fall ist und was am Rohrende zu einem besonders ausgeprägten Effekt führt.

Für Reaktoren mit produktseitiger Thermostatisierung ist gegebenenfalls ein dem oben diskutierten Eduktkreisstrom entsprechender Produktkreisstrom zu installieren, der jedoch nicht, wie weiter oben diskutiert, durch den Katalysator geführt wird, sondern entsprechend dem oben diskutierten Eduktkreisstrom den Produktsammelkanälen zugeführt wird.

Gegebenenfalls können alle drei Varianten zur Verbesserung des Wärmeübergangs gleichzeitig realisiert werden.

Die gezeigten beispielhaften Ausführungsformen machen ferner deutlich, daß in den erfindungsgemäßen Reaktoren über die gesamte Wärmeaustauschfläche eine gleichmäßige Wärmeabgabe beziehungsweise -zufuhr erzielt wird, wobei eine einfachere Reaktorkonstruktion auf der Seite des Thermostatisiermediums ermöglicht wird.

Die erfindungsgemäßen Reaktoren sind dadurch gekennzeichnet, daß das anströmende Edukt auf eine möglichst große Katalysatorbettfläche trifft und gleichzeitig eine möglichst große Fläche zum Wärmeaustausch realisiert wird. Das bedeutet, daß bei einer gegebenen Raum-Zeit-Belastung eine sehr niedrige Aufströmbelastung vorliegt und der Katalysator gleichmäßig temperiert ist. Das wiederum bedeutet, daß in den erfindungsgemäßen Reaktoren, anders als in den bekannten Reaktoren mit kontinuierlicher Thermostatisierung, der Wärmestrom bezüglich des Stoffstroms durch den Katalysator parallel, antiparallel oder parallel und antiparallel geführt. wird. Im Zusammenhang hiermit besitzen die katalytisch aktiven Betten in den erfindungsgemäßen Reaktoren eine besonders geringe Ausdehnung in Strömungsrichtung. Diese bewegt sich bei 0,01-50 cm, bevorzugt bei 0,02-20 cm, besonders bevorzugt bei 0,05-10 cm, ganz besonders bevorzugt bei 0,1-5 cm, ausgesprochen bevorzugt bei 0,2-2 cm. Die Katalysatorschüttung wird in den erfindungsgemäßen Reaktoren durch poröse Wände begrenzt, die neben der Fixierung der Katalysatorschüttung die Aufgabe haben, die gleichmäßige Durchströmung des Katalysatorbetts über die gesamte Oberfläche zu garantieren. Hierzu muß der Strömungswiderstand dieser Wände und des Katalysatorbettes einen gewissen Mindestwert überschreiten, der vom Fachmann leicht experimentell oder durch Rechnung ermittelt werden kann. Im allgemeinen soll der Mindestströmungswiderstand der Katalysatorbetten einschließlich der porösen Gasverteilungswände Werte von 1 mbar bis 10 bar, bevorzugt von 2 mbar bis 1 bar, besonders bevorzugt von 5 mbar bis 500 mbar liegen.

Die porösen Wände in den erfindungsgemäßen Reaktoren bestehen bevorzugt aus Sinterwerkstoffen mit einer entsprechenden Porosität, wie sie zur Gasverteilung dem Fachmann bekannt sind. Besonders bevorzugt sind Sinterformkörper aus Metallen wegen ihrer guten Wärmeleitfähigkeit. Des weiteren sind auch Lochblendenkonstruktionen oder Maschengeflechtkonstruktionen brauchbar. Auch sie müssen die Voraussetzungen erfüllen, den Eduktgasstrom ohne größere örtliche Schwankungen auf den Katalysator zu verteilen und eine gleichmäßige Durchströmung der Schüttung zu gewährleisten; hierzu ist ebenfalls wieder ein genügender Strömungswiderstand erforderlich, der auch mindestens teilweise von der Katalysatorschüttung erzeugt werden kann. Zu diesem Zweck muß das Katalysatormaterial genügend feinkörnig sein, um bei der geringen Durchdringungstiefe den erforderlichen Druckabfall in ausreichender Höhe zu gewährleisten. Es ist ferner verständlich, daß der Katalysator eine ausreichende Rieselfähigkeit haben muß, um beim Vorgang des Einfüllens eine einheitliche Packung zu gewährleisten; so darf das Katalysatormaterial beispielsweise nicht zur Verklebung neigen. Der Zusammenhang zwischen Partikelform und -größe einerseits und dem Strömungswiderstand einer Schüttung aus solchen Partikeln andererseits ist dem Fachmann bekannt und kann experimentell ermittelt werden. Die beiden zur Fixierung der Katalysatorschüttung und zur Gasverteilung dienenden Wände können auch durch einen gegebenenfalls schichtförmigen bzw. aus Schichten aufgebauten katalytisch aktiven Formkörper, der ganz oder teilweise aus Sintermaterial, bevorzugt Sintermetall bestehen kann, ersetzt werden, der gleichzeitig zur Gasverteilung und als Träger für das katalytisch aktive Material dient. Ein solcher Formkörper kann ganz oder teilweise katalytisch aktiv sein. Die prinzipiellen Möglichkeiten zur Belegung von Sinterwerkstoffen mit katalytisch aktiven Partikeln sind dem Fachmann bekannt (EP 416 710).

Ein weiteres Merkmal der erfindungsgemäßen Reaktoren ist ferner die Tatsache, daß der Wärmestrom eine kleine Gasraumstrecke durchqueren muß, um von der Gasverteilungsoberfläche auf der Edukt- und/oder Produktseite zur jeweils infrage kommenden Oberfläche zu gelangen, die das Thermostatisiermedium eingrenzt und selbstverständlich für den Stoffstrom undurchlässig ist; das gleiche gilt für einen Wärmestrom umgekehrt vom Thermostatisiermedium zur Gasverteilungsoberfläche.

Beim Einsatz von umzusetzenden Gasen, die eine geringe Wärmeleitfähigkeit haben, ist es günstig, die Länge des Gasweges zwischen Katalysator und der Trennwand zum Thermostatisiermedium kürzer auszubilden. Es ist zwar prinzipiell möglich, auch bei Gasen mit schlechter Wärmeleitfähigkeit eine große Gaswegstrecke vorzusehen und die Wärmeleitung durch Kühlblechkonstruktionen aus Metall, die von der Trennfläche zum Thermostatisiermedium ausgehen, zu bewirken. Eine solche Konstruktion ist jedoch kompliziert und daher nicht bevorzugt.

Eine weitere Möglichkeit zur Benutzung großer Wegstrecken für die Gase bei schlechter Wärmeleitung ist die Erhöhung der Strömungsgeschwindigkeit, wie dies weiter oben bereits beschrieben wurde und durch einen teilweisen Umlauf des Edukts und/oder des Produkts zum Reaktoreingang und/oder des Produktes in die Sammelkanäle realisiert werden kann. Die Spaltbreite, die der Wärmestrom durch die Gasphase zu überbrücken hat (Abstand zwischen Katalysatorbett und Wandung zum Thermostatisierungsmedium), kann in den erfindungsgemäßen Reaktoren bei 0,1 -10 cm, bevorzugt 0,2 - 8 cm, besonders bevorzugt 0,3 -6 cm, ganz besonders bevorzugt 0,4 - 4 cm liegen.

Der gezielte Einsatz eines durch Wahl der Spaltbreite des Gasraumes festgelegten Wärmeströmungswiderstandes bietet die Möglichkeit, den Temperaturunterschied zwischen dem Thermostatisiermedium und dem Katalysator bei der Auslegung des Reaktors in einen günstigen und gewünschten Bereich zu legen. So ist es beispielsweise möglich, im Zusammenhang mit dem außerordentlich gleichmäßigen Wärmeanfall über die gesamte Austauscherfläche, eine Ölthermostatisierung in den für das gewählte Öl günstigen Temperaturbereich zu legen. Dies hat dann Bedeutung, wenn bei anderer Auslegung des Reaktors von einem Öl als Wärmeträger auf eine komplizierer zu handhabende Salzschmelze übergegangen werden müßte.

Die Temperaturdifferenz zwischen dem Thermostatisiermedium und der Katalysatoroberfläche kann in den erfindungsgemäßen Reaktoren Werte von 2 bis 250°C, bevorzugt 5 bis 200°C, besonders bevorzugt 10 bis 180°C und ganz besonders bevorzugt von 20 bis 130°C annehmen.

Die parallel zur Gasverteilungs- oder Produktsammeloberfläche liegenden Vektorkomponenten der Strömungsgeschwindigkeit der die Wärme transportierenden Gase in den erfindungsgemäßen Reaktoren werden auf Werte von 0 - 1000 m/sec, bevorzugt von 0 - 100 m/sec, besonders bevorzugt 0 - 10 m/sec eingestellt. Der untere Wert Null in diesem Bereich bedeutet hierbei den Betrag den die besagten Vektorkomponenten am Ende der Zuführungsschichten beziehungsweise am Anfang der Sammel- und Ableitungsschichten in Konstruktionen ohne Edukt- beziehungsweise Produktumlauf annehmen.

Die Oberflächenform der Rohre und Gasverteilungselemente kann zur Erzeugung von Turbulenzen von einer glatten Fläche unterschiedlich sein, also beispielsweise Rippen oder ähnliche Zusätze aufweisen.

Für die halbseitig thermostatisierten Reaktortypen gilt, daß die Reaktionsführung in Abhängigkeit von der Bettdicke, der Wärmeleitfähigkeit des Katalysatorbettes, des Gasverteilungssystems und der Gasströme wenig bis teilweise adiabatisch ist. Es werden mehr als 20 %, bevorzugt mehr als 40 %, besonders bevorzugt mehr 60 %, ganz besonders bevorzugt mehr als 80 %, ausgesprochen bevorzugt mehr als 90 % der Reaktionswärme über das Thermostatisiermedium (Wärmeträger) ab- beziehungsweise zugeführt. Zur Verbesserung der Wärmekapazität des umzusetzenden Gasgemisches kann gegebenenfalls diesem eine oder mehrere zusätzliche Wärmeträgerkomponenten zugefügt werden. Diese Komponenten ergeben sich bevorzugt aus den Edukten beziehungsweise Produkten der Umsetzung.

Für die doppelseitig thermostatisierten Reaktortypen gilt, daß die Ausführungsform des Reaktors so gewählt wird, daß der Betrag der Temperaturdifferenz zwischen dem Mittelwert von Ein- und Ausströmtemperatur der Katalysatorbettoberfläche und der Temperatur des Katalysatorbettinneren am heißesten (exotherme Reaktion) beziehungsweise am kältesten (endotherme Reaktion) Punkt zwischen 1 und 300°C, bevorzugt zwischen 1 und 200°C, besonders bevorzugt zwischen 1 und 100°C, ganz besonders bevorzugt zwischen 1 und 60°C, ausgesprochen bevorzugt zwischen 1 und 30°C liegt.

Obengenannte Temperaturdifferenzgrenzen gelten ebenfalls für die halbseitig thermostatisierten Ausführungsformen.

Entsprechend dem Stand der Technik kann ein erfindungsgemäßer Reaktor mit einer druckfesten Ummantelung versehen werden, so daß die in ihm ablaufenden Reaktionen bei über- oder unteratmosphärischem Druck ablaufen können.

Im Unterschied zu den bekannten Druckreaktoren ist es bei erfindungsgemäßen Reaktoren möglich, den Hochdruckteil auf das Zuleitungssystem und das eigentliche Katalysatorbett zu beschränken, indem man Ausströmsinterkörper mit einem entsprechenden Strömungswiderstand verwendet.

Eine derartige Konstruktion bietet den Vorteil, daß der konstruktiv aufwendige Hochdruckraum klein gehalten werden kann.

Bevorzugt werden hierfür z.B. Konstruktionen verwendet, wie sie in Figur 13 und 15 gezeigt sind.

Für Hochdruckreaktionen mit nur teilweisem Umsatz und einer entsprechenden Eduktrecyclisierung ist bei den erfindungsgemäßen Reaktoren hervorzuheben, daß das System auf einen sehr geringen Strömungswiderstand hin optimiert werden kann bei gleichzeitig sehr guter Thermostatisierung, was geringe Pumpleistungen zur Rückführung der komprimierten viskosen Gase zur Folge hat.

Anhand einer Hohlzylinderkonstruktion soll im folgenden auf die Menge der Einbauten in einem solchen Reaktortyp und auf ihre Dimensionierung eingegangen werden. Die Tendenz bei herkömmlichen Rohrbündelreaktoren, zwecks besserer Reaktionskontrolle zu immer kleineren Einbauten überzugehen, seien es nun immer dünnere Rohre oder immer kürzere, führt zu einer Steigerung der Anzahl der Rohre. Technische Reaktoren herkömmlicher Bauart besitzen nicht selten 10.000 Rohre oder mehr. Bei dem in der oben bereits genannten EP 244 632 beschriebenen Kurzrohrreaktor würde sicher eine Anzahl an Rohren von 100.000 erreicht oder überschritten. Am Beispiel von erfindungsgemäßen Rohrbündelreaktoren mit radialem Wärme- und Stofffluß (also parallel oder antiparallel oder sowohl parallel als auch antiparallel) soll verdeutlicht werden, daß mit dem erfindungsgemäßen Reaktorprinzip die Tendenz zu immer zahlreicheren Einbauten durchbrochen wird. Als Vergleichsreaktoren sollen konventionelle Rohrbündelreaktoren mit Rohrinnendurchmessern von 1,5 beziehungsweise 2,5 cm betrachtet werden. Tabelle 1 zeigt die Rohranzahl von erfindungsgemäßen Reaktoren mit einer Bettdicke von 1 cm und unterschiedlichen Rohrdurchmessern, wenn bei gleicher Raumzeitbelastung und Rohrlänge die konventionellen Vergleichsreaktoren 10.000 Rohre beherbergen.

**Tabelle 1**

| Rohranzahl von erfindungsgemäßen Rohrbündelreaktoren im Vergleich zu konventionellen Rohrbündelreaktoren (ø bedeutet Durchmesser) | | | |
|---|---|---|---|
| konventionelle Rohrbündelreaktoren (10.000 Rohre) | erfindungsgemäße Rohrbündelreaktoren | | |
| | ø = 40 | ø = 20 | ø = 10 cm |
| | Zahl der Rohre | | |
| ø = 1,5 cm | 145 | 294 | 625 |
| ø = 2,5 cm | 400 | 833 | 1666 |

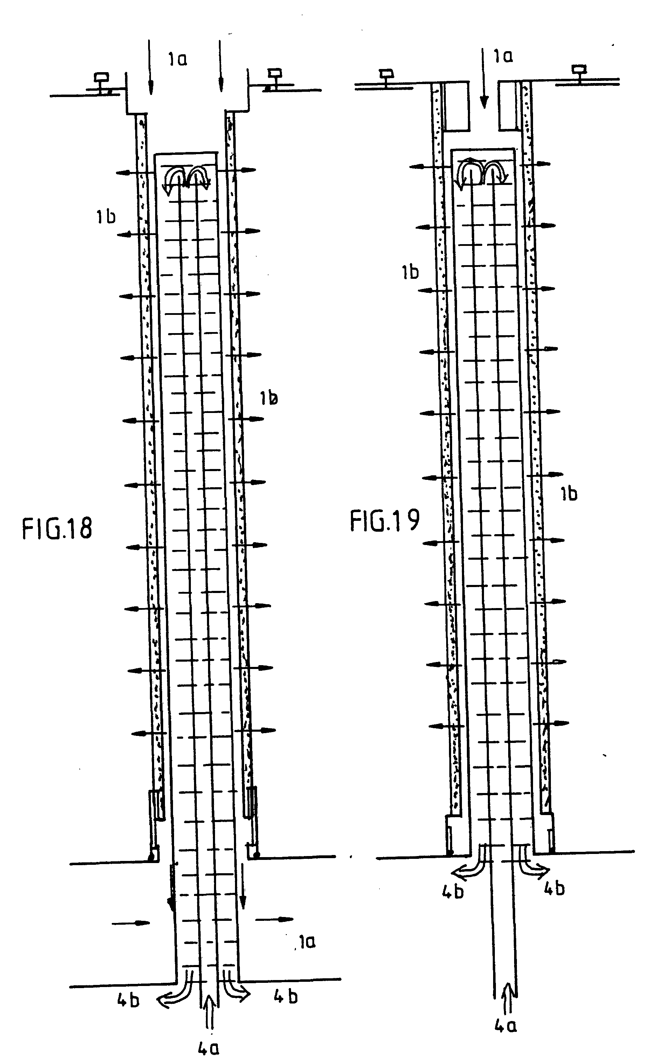

Erfindungsgemäße Reaktorrohre mit 40 cm ø können beispielsweise solche wie in Figur 18 sein, wobei das dünnschichtige Katalysatorbett als Hohlrohr ausgebildet ist und über einen Zapfen für den Durchlauf des Wärmeträgermediums geschraubt wird. Erfindungsgemäße Reaktorrohre mit 10 cm ø sind beispielsweise solche der in Figur 16 gezeigten Art, wobei die dünnschichtige Katalysatorschicht wiederum als Hohlrohr ausgebildet ist, aber im Gegensatz zu Figur 18 in eine entsprechende Aussparung innerhalb des vom Wärmeträgermediums durchflossenen Volumens hineingeschraubt wird. Allgemein und unabhängig von der beispielhaften Angabe in der obigen Tabelle 1 liegen die Rohrdurchmesser der erfindungsgemäßen Rohrbündelreaktoren mit hohlzylinderförmiger Katalysatoranordnung im Bereich von 5 - 200 cm, bevorzugt von 7 - 100 cm, besonders bevorzugt von 10 -50 cm. Die Rohrlängen bewegen sich im Bereich 1-40 m, bevorzugt von 2-20 m, besonders bevorzugt von 3-10 m. In Anlehnung an bekannte Bauformen werden auch die erfindungsgemäßen Katalysator-Hohlzylinder im allgemeinen zu mehreren von ihnen als größeres Aggregat, beispielsweise als Rohrbündel, eingesetzt.

Die erfindungsgemäßen Reaktoren können prinzipiell für jede heterogen katalysierte Gasphasenreaktion eingesetzt werden. Besonders geeignet sind sie jedoch für Reaktionen, die zu einem ausgeprägten hot-spot in konventionelle Rohrreaktoren neigen, weiterhin für Reaktionen, die in einem eng begrenzten Temperaturbereich ablaufen müssen, weiterhin für Reaktionen, deren Katalysator rasch Überlastet ist und dann zu Nebenreaktionen neigt, weiterhin für Reaktionen, die eine genau definierte Verweilzeit erfordern, da sie sonst zu Folgereaktionen neigen und schließlich für Reaktionen, die extrem kurze Verweilzeiten bei gleichzeitig guter Kühlung benötigen, weil sie extrem rasch ablaufen und dann zu Folgereaktionen neigen.

Die Nebenprodukte und Folgereaktionsprodukte einer Gasphasenumsetzung, die bedingt sind durch die Temperatur, die Katalysatorbelastung oder die Verweilzeit, können entscheidend zur Desaktivierung des Katalysators durch Koksbildung beitragen. Dies ist im Grunde eine ähnlich unerwünschte Desaktivierung wie die Katalysatordesaktivierung durch reine Temperaturüberlastung. Daher sind die erfindungsgemäßen Reaktoren besonders gut geeignet, wenn lange Katalysatorstandzeiten erfordert sind, sei es, weil der Katalysator nicht regenerierbar ist oder weil die technische Umsetzung möglichst wenig wartungsintensiv sein muß. Die exakte Reaktionsführung und die besonders geringe Desaktivierungsneigung der Katalysatoren in den erfindungsgemäßen Reaktoren läßt sich durch die geringe Durchdringungstiefe der Katalysatorbetten erklären und dadurch, daß schwerflüchtige Koksvorläufer das Katalysatorbett verlassen können, ehe sie stationär geworden sind.

Beispiele für besonders günstig durchführbare Reaktionen in den erfindungsgemäßen Reaktoren sind, ohne das diese Aufzählung erschöpfend sind: Dehydrierungen z.B. von Cyclohexyliden-anilin zu Diphenylamin und von Cyclohexenyl-cyclohexanon zu o-Phenylphenol; Hydrierungen z.B. von Nitrobenzol zu Anilin und von Phenol zu Cyclohexanon; oxidative Alkoholdehydrierungen z.B. von 3-Methyl-3-buten-1-ol zu 3-Methyl-2-buten-1-al; Selektivoxidationen von Kohlenwasserstoffen z.B. von n-Butan zu Maleinsäureanhydrid, von o-Xylol zu Phthalsäureanhydrid und von Propen zu Acrolein beziehungsweise Acrylsäure sowie Alkylierungen und Halogenierungen.

Das außergewöhnlich erfolgreiche Funktionieren der erfindungsgemäßen Reaktoren ist überraschend, da nach dem ingenieurtechnischen Wissensstand Radialreaktoren, wenn überhaupt, nur sehr kompliziert zu thermostatisieren sind (Chem. Tech. 30 (1978), 74). Dieses Vorurteil im Rahmen des ingenieurtechnischen Wissensstandes gründet sich vor allem auf die schlechte Wärmeleitfähigkeit von Gasen. Die erfindungsgemäßen Reaktoren sollten nach diesem Wissensstand daher nur für adiabatisch zu führende Reaktionen geeignet sein.

### Beispiel 1

### Herstellung des Katalysators für die Dehydrierung von Cyclohexyliden-anilin zu Diphenylamin

Ein Liter eines Aluminiumoxidträgers, bestehend aus Kugeln mit einem Durchmesser von 2,0 bis 3,0 mm, einer BET-Oberfläche von 74 m²/g, einer Saugfähigkeit von 54 ml H₂O pro 100 g Träger und einem Schüttgewicht von 717 g/l, wurde mit 387 ml einer wäßrigen NaOH-Lösung imprägniert, die 13,5 g, entsprechend 0,34 Grammäquivalent NaOH enthielt. Die Lösung wurde innerhalb von 2 Minuten völlig im Träger aufgesaugt. Der feuchte Träger wurde in ein senkrechtes Glasrohr von ca. 2 l Inhalt geschüttet und dann mit warmer Luft von 105 bis 120° C in einer Menge von 20 m³ /h von unten nach oben behandelt. Es wurde bis zur Gewichtskonstanz des Trägers getrocknet, was nach 30 Minuten der Fall war. Der Restfeuchtegehalt des getrockneten Trägers lag nach Abkühlen auf Raumtemperatur bei 0,25 Gew.-%, entsprechend ca. 0,47 % der Saugfähigkeit.

Der vorbehandelte, trockene Träger wurde seiner Saugfähigkeit entsprechend mit einer wäßrigen Natriumtetrachloropalladat-Lösung, 18 g Pd, entsprechend 0,216 Grammäquivalent enthaltend, getränkt und 15 Minuten stehengelassen. Dann wurde der Katalysator in einem fließenden Strom von destilliertem Wasser gewaschen bis keine Natrium- und Chloridionen im Waschwasser mehr nachweisbar waren, was nach 12 Stunden der Fall war.

Die anschließende Trocknung geschah im Warmluftstrom, wie vorher bei der Trocknung des Trägers beschrieben. Der so hergestellte Katalysator enthielt, als Metall berechnet, 18 g Pd pro Liter Träger ( 1,555 Gew.-%).

### Beispiel 2

### Dehydrierung von Cyclohexyliden-anilin zu Diphenylamin in einem Rohreaktor (Vergleichsbeispiel)

In die Mitte eines ölthermostatisierten Glasrohrreaktors (Länge = 40 cm, Innendurchmesser = 2,3 cm) wurde eine 15 cm lange Katalysatorschüttung eingebracht.

Die Katalysatorschüttung befand sich zwischen zwei Schüttungen aus 5 mm Glaskugeln. Der Ölmantel des Reaktors wurde mit einem Hochtemperaturöltermostat verbunden und der Katalysator in einem Wasserstoffstrom auf 250°C aufgeheizt. Der Wasserstoffstrom wurde durch einen Stickstoffstrom ersetzt und die Temperatur des Ölmantels auf 320°C heraufgesetzt. Mit einer Belastung von 0,3 g/ml·h (bezogen auf das Katalysator-Schüttungsvolumen) wurde Cyclohexyliden-anilin in einem Stickstoffstrom über den Kontakt geleitet (1 l N₂/g Cyclohexyliden-anilin). Bei 100%igem Umsatz verließ mit 95%iger Selektivität Diphenylamin in einer Stickstoff-Wasserstoff-Mischung gasförmig den Reaktor.

Die Hauptumsatzzone gab sich im Reaktorbett durch ein Temperaturtal zu erkennen. Dieses Temperaturtal bewegte sich im Laufe der Zeit vom Katalysatorkopf in Richtung Katalysatorende. Nach ca. 100 bis 150 Stunden machte sich die fortschreitende Katalysatordesaktivierung durch einen rasch steigenden Cyclohexyliden-anilin-Anteil im Produktgemisch bemerkbar.

### Beispiel 3

### Dehydrierung von Cyclohexyliden-anilin zu Diphenylamin in einem Hohlzylinderrohrreaktor gemäß Erfindung

Der Reaktor bestand aus einem ölthermostatisierten Glasrohr (Länge 62 cm, Innendurchmesser 4,9 cm). Konzentrisch zu diesem Glasrohr befand sich ein Rohr aus Glassinterwerkstoff im Innern des themostatisierten Glasrohrs. Dieses Rohr (Länge = 15 cm, Durchmesser = 2,0 cm, Porosität 3) war am oberen Ende zugeschmolzen und am unteren Ende an ein evakuiertes Doppelmantelglasrohr mit einem Kegelglasschliff (Normalschliff NS29) angeschmolzen (inneres Glasrohr vom Durchmesser 2,4 cm, äußeres Glasrohr von Durchmesser 3,8 cm); der Raum zwischen innerem und äußeren Glasrohr war evakuiert. Durch das innere Glasrohr wurde das Produktgas abgeführt. Das äußere Glasrohr besaß, gemessen vom NS29-Kegelschliff bis zum unteren Ende des thermostatisierten Glasrohrs, eine Länge von 25 cm. Am Ende des thermostatisierten Glasrohres schloß das äußere Glasrohr mit dem thermostatisierten Glasrohr gasdicht ab. Konzentrisch zum inneren Glassinterrohr befand sich ein weiteres Glassinterrohr im Innern des ölthermostatisierten Glasrohr (Länge = 15 cm, Innendurchmesser = 3,2 cm, Wandstärke = 2 mm, Porosität 3). Dieses Glassinterrohr war am unteren Ende an eine NS29-Glasschliffhülse angeschmolzen, welche auf dem Glasschliffkern des Vakuumdoppelmantelrohres gasdicht aufsaß. Das äußere Glassinterrohr war am oberen Ende mit einem 10 cm langen Glasrohr mit NS29-Hülse verschmolzen. In dieser Hülse saß gasdicht ein ca. 10 cm langer evakuierter Glasschliffstopfen.

Zwischen dem äußeren und inneren Glassinterrohr befand sich die Katalysatorschüttung.

Der Ölmantel des Reaktors wurde mit einem Hochtemperaturölthermostat verbunden und der Katalysator in einem Wasserstoffstrom auf 250°C aufgeheizt. Der Wasserstoffstrom wurde durch einen Stickstoffstrom ersetzt und die Temperatur des Ölmantels auf 320°C heraufgesetzt. Mit einer Belastung von 0,3 g/ml·h (bezogen auf das Katalysatorvolumen) wurde Cyclohexyliden-anilin in einem Stickstoffstrom von außen nach innen über den Kontakt geleitet (1 l N₂/g Cyclohexyliden-anilin). Bei 100%igem Umsatz verließ mit 95%iger Selektivität Diphenylamin in einer Stickstoff-Wasserstoff-Mischung gasförmig den Reaktor. Der Versuch wurde nach 1000 Stunden Laufzeit ohne nennenswerte Änderungen in der Produktzusammensetzung (d.h. ohne Katalysatordesaktivierung) beendet.

### Beispiel 4

### Herstellung des Katalysators für die Nitrobenzolreduktion zu Anilin

Ein Liter eines α-Aluminiumoxidträgers in Kugelform mit 3 bis 4 mm Durchmesser, einer BET-Oberfläche von 9,8 m² /g, einer Saugfähigkeit von 45,1 ml Wasser pro 100 g Träger und einem Schüttgewicht von 812 g/l wurde mit 366 ml einer 10,8 g, entsprechend 0,27 Grammäquivalenten, NaOH enthaltenden wässrigen Lösung imprägniert. Die Lösung wurde innerhalb weniger Minuten völlig vom Träger aufgenommen. Der feuchte Träger wurde in ein senkrechtes Glasrohr von ca. 2 l Inhalt geschüttet und im Warmluftstrom von 120°C bei einer Luftmenge von 25 Nm³ Luft pro Stunde getrocknet. Die Trockenzeit bis zur Gewichtskonstanz betrug etwa 30 Minuten. Der Restfeuchtegehalt lag nach dem Abkühlen auf Raumtemperatur bei ca. 0,9 % der Saugfähigkeit des Trägers.

Der so vorbehandelte trockene Träger wurde, seiner Saugfähigkeit entsprechend, mit 366 ml einer wässrigen Natriumtetrachlorpalladat-(II)-Lösung, die 9 g Palladium, entsprechend 0,169 Grammäquivalent, enthielt, getränkt und 15 Minuten stehen gelassen. Zur Reduktion der auf dem Träger abgeschiedenen Palladiumverbindung zu metallischen Palladium wurde der Träger in einem Becherglas mit 400 ml einer 10%igen wässrigen Hydrazinhydrat-Lösung überschichtet und 2 Stunden stehen gelassen. Danach wurde der Katalysator in einem fließenden Strom von destilliertem Wasser gewaschen, bis keine Ionen der bei der Katalysatorherstellung verwendeten Verbindungen im Waschwasser mehr nachweisbar waren, was nach 10 Stunden der Fall war.

Die anschließende Trocknung wurde im Warmluftstrom vorgenommen, wie vorher bei der Trocknung des Trägers beschrieben. Der so hergestellte Katalysator enthielt 9 g Palladium pro Liter Träger.

Der Pd-haltige Katalysator wurde anschließend mit 366 ml einer wäßrigen, 9 g Vanadium in Form von Vanadyloxalat enthaltenden Lösung getränkt. Die Trocknung des mit Vanadyloxalat imprägnierten Pd-Katalysators erfolgte analog der Trocknung des Trägers im Warmluftstrom bei 120°C. Die anschließende Zersetzung des Vanadyloxalates wurde während 6 Stunden bei 300°C durchgeführt.

Nach dieser Behandlung wurde der Katalysator mit 366 ml einer wäßrigen, 3 g Blei in Form von Bleiacetat enthaltenden Lösung getränkt. Der mit Bleiacetat imprägnierte Katalysator wurde in feuchtem Zustand in einen Rohrreaktor gefüllt und während der Aufheizperiode des Wärmeträgers getrocknet.

Die Aktivierung des Katalysators wurde im Wasserstoffstrom bei der Temperatur des Wärmeträgers, die 280°C betrug, durchgeführt. Der fertige Katalysator enthielt, als Metall berechnet, 9 g Palladium, 9 g Vanadium und 3 g Blei pro Liter Träger.

### Beispiel 5

### Nitrobenzolhydrierung in einem Rohrreaktor (Vergleichsbeispiel)

Ein 200 cm langer ölthermostatisierter Metallrohrreaktor wurde mit einer 150 cm langen Katalysatorschüttung versehen (Durchmesser 2,3 cm). Bei 200°C Öltemperatur wurde ein Wasserstoffstrom über den Katalysator geleitet (594.9 Nl/Std.), die Öltemperatur auf 270°C heraufgenommen und eine Katalysatorbelastung von 0,5 g Nitrobenzol pro ml Katalysator und Stunde eingestellt (Belastung = 0,5 g/ml h, H₂-Überschuß 250 %).

In der Nähe des Katalysatorkopfes bildete sich eine heiße Zone mit einer Temperatur von ca. 450°C aus, die sich mit annähernd konstanter Geschwindigkeit in Richtung Katalysatorende bewegte.

Nach etwa 1000 Stunden Laufzeit erreichte diese Zone das Katalysatorende, worauf mit rasch steigender Intensität Nitrobenzol im Produktgasgemisch nachzuweisen war. Bevor die fortschreitende Desaktivierung das Katalysatorende erreichte, wurde bei 100%igem Umsatz mit einer Selektivität besser 99,5 % Anilin gebildet.

### Beispiel 6

### Nitrobenzolhydrierung in einem Kurzrohrreaktor (Vergleichsbeispiel)

In der Mitte des in Beispiel 2 beschriebenen Glasreaktors wurde eine 15 cm lange Katalysatorschüttung plaziert. Nach der Aktivierung im Wasserstoffstrom wurde bei einer Ölbadtemperatur von 220°C Nitrobenzol mit einer Belastung von 0,5 g/ml h bei einem Wasserstoffüberschuß von 250 % über den Kontakt geleitet. Am Katalysator bildete sich eine heiße Zone mit einer Maximaltemperatur von ca. 360°C aus. Diese heiße Zone bewegte sich unter Abflachung auf das Katalysatorende zu. Nach ca. 1000 Stunden Laufzeit begann Nitrobenzol den Reaktor mit dem Produktgasgemisch zu verlassen. Bevor die Desaktivierung das Katalysatorende erreichte, wurde bei 100%igem Umsatz Anilin mit einer Selektivität von besser als 99,5 % gebildet.

### Beispiel 7

### Nitrobenzolhydrierung in einem Hohlzylinderrohrreaktor gemäß Erfindung

Der in Beispiel 3 beschriebene Hohlzylinderreaktor erhielt im Austausch einen anderen Vakuum-Doppelmantel-Einsatz mit angeschmolzenem Glassinterhohlzylinder. Länge des am oberen Ende zugeschmolzen Glassinterrohres 15 cm, Durchmesser 1,2 cm. Die radial zu durchströmende Bettdicke stieg damit von 6 mm auf 10 mm. Der Raum zwischen den Glassinterrohren wurde mit Katalysator gefüllt.

Wie in Beispiel 5 und Beispiel 6 wurde der Katalysator nach einer Vorreduktion bei 220°C Ölbadtemperatur einer Belastung von 0,5 g Nitrobenzol pro ml Katalysator und Stunde bei 250%igem Wasserstoffüberschuß betrieben. Im Innern des inneren Glassinterrohrs wurde mittels eines Temperaturmeßfühlers eine Temperatur von 320°C gemessen. Der Versuch wurde nach 4000 Stunden Laufzeit ohne nennenswerte Katalysatordesaktivierung beendet. Während dieses Zeitraumes wurde Nitrobenzol zu 100 % umgesetzt und Anilin mit mehr als 99,8%iger Selektivität gebildet.

### Beispiel 8 (zum Vergleich)

### Nitrobenzolhydrierung in einem Hohlzylinderreaktor

Der Versuch aus Beispiel 7 wurde bei 320°C Öltemperatur wiederholt. Die Austrittstemperatur des Produktgasgemisches im Innern des Reaktors betrug 430°C. Nach 600 Stunden Laufzeit waren Produktzusammensetzung und Austrittstemperatur unverändert (Umsatz 100 %, Selektivität > 99,8 %); erst nach 800 Stunden Laufzeit zeigte sich eine beginnende Katalysatordesaktivierung.

## Patentansprüche

1. Reaktoren für die kontinuierliche Umsetzung gasförmiger Stoffe an Festbettkatalysatoren bei kontinuierlicher Temperaturlenkung mit Hilfe eines Wärmeträgers, **gekennzeichnet durch** die Ausbildung des Katalysatorbettes in Form von einer oder mehreren regelmäßig geformten flächigen Schichten mit einer Dicke von 0,01 - 50 cm, wobei die Oberfläche der Katalysatorschichten durch eine für Gase durchlässige Schicht abgeschlossen ist und wobei diese Oberfläche auf der Eduktanströmseite und/oder auf der Produktaustrittsseite in einem Abstand von 0,1 - 10 cm einer in gleicher Weise regelmäßig geformten Wandung gegenüber steht, die den Raum für die umzusetzenden Stoffe beziehungsweise umgesetzten Stoffe vom Raum für den Wärmeträger trennt, und **gekennzeichnet durch** eine solche Führung der umzusetzenden Stoffe, daß sie das Katalysatorbett senkrecht zu den flächigen Katalysatorschichten durchströmen.

2. Reaktor nach Anspruch 1, **dadurch gekennzeichnet, daß** das Katalysatorbett eine Dicke von 0,02 - 20 cm, bevorzugt 0,05 - 10 cm, besonders bevorzugt 0,1 - 5 cm, ganz besonders bevorzugt 0,2-2 cm hat.

3. Reaktor nach Anspruch 1, **dadurch gekennzeichnet, daß** der Abstand zwischen Katalysatorbett und Wandung 0,2 - 8 cm, bevorzugt 0,3 - 6 cm, besonders bevorzugt 0,4 - 4 cm beträgt.

4. Reaktor nach Anspruch 1, **dadurch gekennzeichnet, daß** die für Gase durchlässige Schicht zum Abschluß der Katalysatorschichten als Maschenwerk, Lochblende oder Sintermaterial, bevorzugt als Sintermaterial, besonders bevorzugt als Sintermetall ausgebildet ist.

5. Reaktor nach Anspruch 1, **dadurch gekennzeichnet, daß** das flächige Katalysatorbett einschließlich seiner für Gase durchlässige abschließende Schicht einen Mindestströmungswiderstand von 1 mbar bis 10 bar, bevorzugt von 2 mbar bis 1 bar, besonders bevorzugt von 5 - 500 mbar hat.

6. Reaktor nach Anspruch 1, **dadurch gekennzeichnet, daß** die regelmäßig geformten flächigen Schichten des Katalysatorbettes als einseitig abgeschlossene Hohlzylinder mit Durchmessern von 5 - 200 cm, bevorzugt 7 - 100 cm, besonders bevorzugt 10 -50 cm ausgebildet sind, die bevorzugt zu mehreren von ihnen als größeres Aggregat, besonders bevorzugt als Rohrbündel angeordnet sind.

7. Verfahren zur katalysierten, thermogelenkten Gasphasenreaktion mit hoher endothermer oder exothermer Wärmetönung, **dadurch gekennzeichnet, daß** ein Reaktor nach Anspruch 1 eingesetzt wird.

8. Verfahren nach Anspruch 7, **dadurch gekennzeichnet, daß** es sich um Dehydrierungen, Hydrierungen, Oxidationen, Alkylierungen oder Halogenierungen handelt.

## Claims

1. Reactors for the continuous reaction of gaseous substances on solid bed catalysts with continuous temperature control by means of a heat transfer medium, **characterised by** the design of the catalyst bed in the form of one or more regularly shaped flat layers with a thickness from 0.01 cm to 50 cm, the surface of the catalyst layers being sealed by a gas permeable layer and said surface on the starting product inflow side and/or on the product outlet side, at a distance of 0.1 cm to 10 cm, being situated opposite a similarly regularly shaped wall which separates the compartment for the substances to be reacted or which have reacted from the compartment for the heat transfer medium, and **characterised in that** the substances to be reacted are directed such that they flow through the catalyst bed perpendicularly to the flat catalyst layers.

2. A reactor according to claim 1, **characterised in that** the catalyst bed has a thickness from 0.02 cm to 20 cm, preferably 0.05 cm to 10 cm, particularly preferably 0.1 cm to 5 cm, more particularly preferably 0.2 cm to 2 cm.

3. A reactor according to claim 1, **characterised in that** the distance between catalyst bed and wall is 0.2 cm to 8 cm, preferably 0.3 cm to 6 cm, particularly preferably 0.4 cm to 4 cm.

4. A reactor according to claim 1, **characterised in that** the gas permeable layer for sealing the catalyst layers takes the form of a meshwork, aperture plate or sintered material, preferably a sintered material, particularly preferably sintered metal.

5. A reactor according to claim 1, **characterised in that** the flat catalyst bed including its gas permeable sealing layer has a minimum flow resistance from 1 mbar to 10 bar, preferably from 2 mbar to 1 bar, particularly preferably from 5 mbar to 500 mbar.

6. A reactor according to claim 1, **characterised in that** the regularly shaped flat layers of the catalyst bed take the form of hollow cylinders sealed on one side, with diameters from 5 cm to 200 cm, preferably 7 cm to 100 cm, particularly preferably 10 cm to 50 cm, several of which are arranged preferably as a relatively large assembly, particularly preferably as a bundle of tubes.

7. Processes for the catalysed, temperature-controlled gas phase reaction with a high endothermic or exothermic heat effect, **characterised in that** a reactor according to claim 1 is used.

8. Processes according to claim 7, **characterised in that** they are dehydrogenation, hydrogenation, oxidation, alkylation or halogenation processes.

## Revendications

1. Réacteurs pour la conversion continue de substances gazeuses sur des catalyseurs en lit fixe avec une commande continue de la température au moyen d'un caloporteur, **caractérisés par** l'agencement du lit de catalyseur sous forme d'une ou plusieurs couches à grande surface formées régulièrement ayant une épaisseur de 0,01-50 cm, où la surface des couches de catalyseur est fermée par une couche perméable aux gaz et où cette surface, du côté d'afflux des éduits et/ou du côté de sortie des produits, est située en face, à une distance de 0,1-10 cm, d'une paroi formée régulièrement de la même manière qui sépare l'espace pour les substances à convertir ou les substances converties de l'espace pour le caloporteur, et **caractérisés par** une conduite des substances à convertir telle qu'elles traversent le lit de catalyseur perpendiculairement aux couches de catalyseur à grande surface.

2. Réacteur selon la revendication 1, **caractérisé en ce que** le lit de catalyseur a une épaisseur de 0,02-20 cm, de préférence de 0,05-10 cm, de préférence encore de 0,1-5 cm et de manière particulièrement préférée de 0,2-2 cm.

3. Réacteur selon la revendication 1, **caractérisé en ce que** la distance entre le lit de catalyseur et la paroi est de 0,2-8 cm, de préférence de 0,3-6 cm, de préférence encore de 0,4-4 cm.

4. Réacteur selon la revendication 1, **caractérisé en ce que** la couche perméable aux gaz pour fermer les couches de catalyseur est sous forme de treillis à mailles, de diaphragme perforé ou de matériau fritté, de préférence sous forme de matériau fritté, de manière particulièrement préférée sous forme de métal fritté.

5. Réacteur selon la revendication 1, **caractérisé en ce que** le lit de catalyseur à grande surface y compris sa couche de fermeture perméable aux gaz a une résistance à l'écoulement minimale de 1 mbar à 10 bar, de préférence de 2 mbar à 1 bar, de manière particulièrement préférée de 5-500 mbar.

6. Réacteur selon la revendication 1, **caractérisé en ce que** les couches à grande surface du lit de catalyseur qui sont formées régulièrement sont sous forme de cylindres creux fermés d'un côté ayant des diamètres de 5-200 cm, de préférence de 7-100 cm, de manière particulièrement préférée de 10-50 cm, qui forment de préférence à plusieurs une unité de plus grande taille, de manière particulièrement préférée un faisceau de tubes.

7. Procédé de réaction en phase gazeuse thermocommandée, catalysée, à grande chaleur de réaction endothermique ou exothermique, **caractérisé en ce qu'**un réacteur selon la revendication 1 est utilisé.

8. Procédé selon la revendication 7, **caractérisé en ce qu'**il s'agit de déshydrogénations, d'hydrogénations, d'oxydations, d'alkylations ou d'halogénations.
